(19) Europäisches Patentamt
European Patent Office
Office européen des brevets

(11) EP 2 885 274 B1

(12) EUROPEAN PATENT SPECIFICATION

(45) Date of publication and mention of the grant of the patent:
**03.10.2018 Bulletin 2018/40**

(21) Application number: **13750644.0**

(22) Date of filing: **20.08.2013**

(51) Int Cl.:
***C07D 209/86*** (2006.01) ***C07D 209/88*** (2006.01)
***A61P 31/04*** (2006.01) ***A61K 31/403*** (2006.01)
***C12N 1/20*** (2006.01) ***C12R 1/47*** (2006.01)
***C12P 17/10*** (2006.01)

(86) International application number:
**PCT/EP2013/002507**

(87) International publication number:
**WO 2014/029498 (27.02.2014 Gazette 2014/09)**

# (54) BIXIAMYCINS AND SULFONYLBIXIAMYCINS

BIXIAMYCINE UND SULFONYLBIXIAMYCINE

BIXIAMYCINES ET SULFONYLBIXIAMYCINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2012 US 201261684941 P**

(43) Date of publication of application:
**24.06.2015 Bulletin 2015/26**

(73) Proprietor: **Leibniz-Institut für Naturstoff-Forschung und Infektionsbiologie Hans Knöll Institut**
**07745 Jena (DE)**

(72) Inventors:
- **HERTWECK, Christian**
  **07745 Jena (DE)**
- **DING, Ling**
  **07745 Jena (DE)**
- **XU, Zhongli**
  **07745 Jena (DE)**
- **BAUNACH, Martin**
  **07745 Jena (DE)**

(74) Representative: **Forstmeyer, Dietmar et al**
**BOETERS & LIECK**
**Oberanger 32**
**80331 München (DE)**

(56) References cited:
- **QINGBO ZHANG ET AL: "N-N-Coupled Indolo-sesquiterpene Atropo-Diastereomers from a Marine-Derived Actinomycete", EUROPEAN JOURNAL OF ORGANIC CHEMISTRY, vol. 2012, no. 27, 13 August 2012 (2012-08-13), pages 5256-5262, XP055080779, ISSN: 1434-193X, DOI: 10.1002/ejoc.201200599**
- **ZHONGLI XU ET AL: "Bacterial Synthesis of Diverse Indole Terpene Alkaloids by an Unparalleled Cyclization Sequence", ANGEWANDTE CHEMIE INTERNATIONAL EDITION, vol. 51, no. 41, 8 October 2012 (2012-10-08), pages 10293-10297, XP055080776, ISSN: 1433-7851, DOI: 10.1002/anie.201204087**
- **Martin Baunach ET AL: "Regiodivergent NC and NN Aryl Coupling Reactions of Indoloterpenes and Cycloether Formation Mediated by a Single Bacterial Flavoenzyme", Angewandte Chemie International Edition, vol. 52, no. 34, 19 August 2013 (2013-08-19), pages 9040-9043, XP055262193, DE ISSN: 1433-7851, DOI: 10.1002/anie.201303733**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 885 274 B1

## Description

The present invention provides novel dimeric and sulfonyl dimeric indolosesquiterpenes having antibacterial activities. These compounds can e.g. be isolated from a mangrove endophyte.

Indole terpenoids encompass a highly diverse group of natural products, including the anticancer agents vincristine and vinblastine, psychotropic agents such as lysergic acid derivatives, the aphrodisiac yohimbine, and the potassium channel blockers paxilline and lolitrem.

In light of the impressive number of known eukaryotic indole terpene metabolites, it is remarkable that only recently the first representatives of this group derived from bacteria were discovered, including oridamycin, xiamycin, indosespene, and sespenine (Takada 2010, Ding 2010, Ding 2011). Xiamycin (1) was produced by two *Streptomyces* endophytes (HKI0576 and HKI0595) of widespread mangrove trees, *Bruguiera gymnorrhiza* and *Kandelia candel,* respectively (Ding 2010, Ding 2011).

From the two strains, the dimers of xiamycin have now been detected. Heterologous expression of the xiamycin biosynthesis gene cluster (Li 2012) in *S. albus,* follow-up fermentation and isolation yielded various *N-N,* and *C-N* dimers as well as sulfonyl bridged dimers of xiamycins (**2a, 2b, 3a, 3b, 4, 5, 6,** and **7**). They all exhibit antibacterial activities and no cytotoxic effects indicating their use as antibacterial agents. Figure 1 shows the structures of xiamycin, bixiamycins and sulfonylbixiamycins.

The present invention provides a compound of formula (II)

X-A-Y (II)

wherein A is a bond, a sulphur atom or a group of formula -SO- or $-SO_2-$,
wherein X is selected from the following structures:

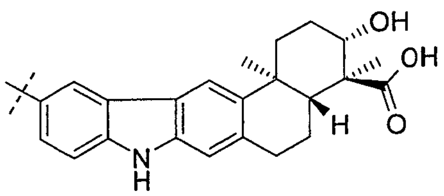

or

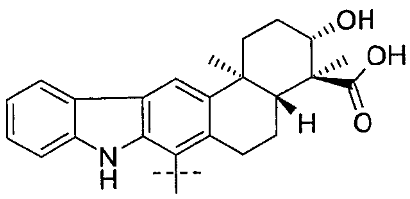

and wherein Y has the following structure of formula (I) wherein one hydrogen atom is replaced by the bond to A or X

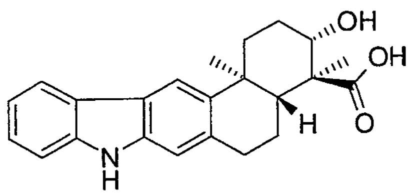

(I)

or a pharmaceutically acceptable salt, solvate or hydrate or a pharmaceutically acceptable formulation thereof.

Preferably, Y is selected from the following structures:

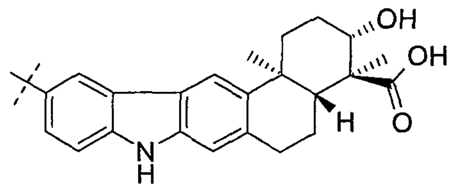

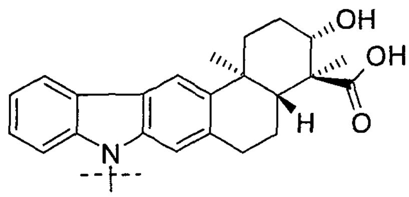

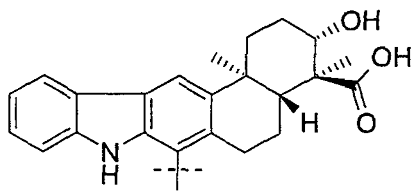

Especially preferred are the following compounds:

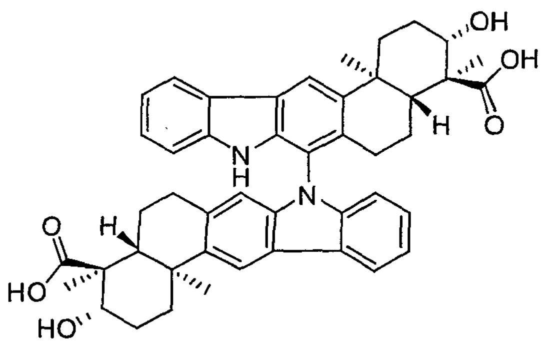

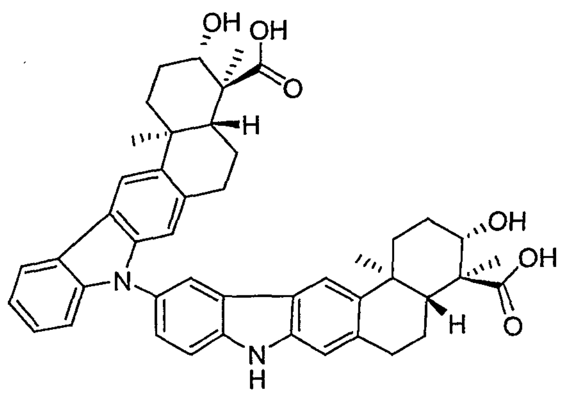

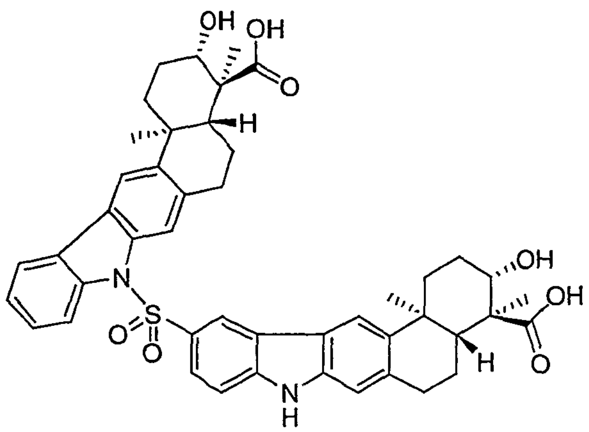

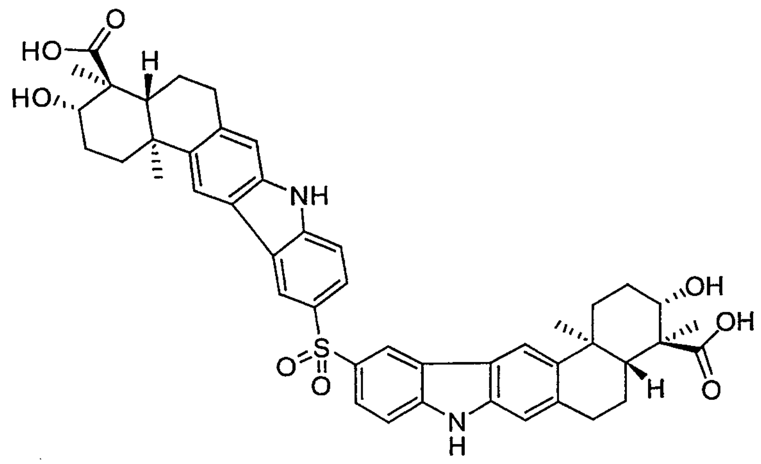

and

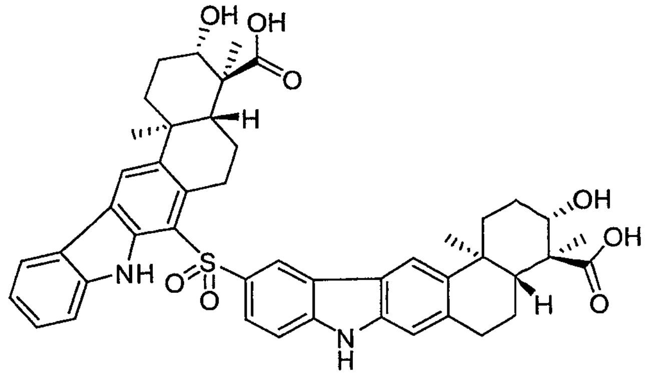

The present invention further provides pharmaceutical compositions comprising one or more compounds described herein or a pharmaceutically acceptable salt, solvate or hydrate thereof, optionally in combination with a pharmaceutically acceptable carrier.

It is a further object of the present invention to provide a compound as described herein or a pharmaceutical composition as defined herein for the preparation of a medicament for the treatment and/or prophylaxis of bacterial infections.

Examples of pharmacologically acceptable salts of sufficiently basic compounds are salts of physiologically acceptable mineral acids like hydrochloric, hydrobromic, sulfuric and phosphoric acid; or salts of organic acids like methanesulfonic, p-toluenesulfonic, lactic, acetic, trifluoroacetic, citric, succinic, fumaric, maleic and salicylic acid. Further, a sufficiently acidic compound may form alkali or earth alkali metal salts, for example sodium, potassium, lithium, calcium or magnesium salts; ammonium salts; or organic base salts, for example methylamine, dimethylamine, trimethylamine, triethylamine, ethylenediamine, ethanolamine, choline hydroxide, meglumin, piperidine, morpholine, tris-(2-hydroxyethyl)amine, lysine or arginine salts; all of which are also further examples of salts of the compounds described herein. The compounds described herein may be solvated, especially hydrated. The hydratization/hydration may occur during the process of production or as a consequence of the hygroscopic nature of the initially water free compounds. The solvates and/or hydrates may e.g. be present in solid or liquid form.

The therapeutic use of the compounds described herein, their pharmacologically acceptable salts, solvates and hydrates, respectively, as well as formulations and pharmaceutical compositions also lie within the scope of the present invention.

The pharmaceutical compositions according to the present invention comprise at least one compound described herein and, optionally, carrier substances and/or adjuvants.

The pharmaceutical composition may optionally comprise one or more further antibacterial compounds or may be administered in combination with one or more further antibacterial compounds.

As mentioned above, therapeutically useful agents that contain compounds described herein, their solvates, salts or formulations are also comprised in the scope of the present invention. In general, the compounds described herein will be administered by using the known and acceptable modes known in the art, either alone or in combination with any other therapeutic agent.

For oral administration such therapeutically useful agents can be administered by one of the following routes: oral, e.g. as tablets, dragees, coated tablets, pills, semisolids, soft or hard capsules, for example soft and hard gelatine capsules, aqueous or oily solutions, emulsions, suspensions or syrups, parenteral including intravenous, intramuscular and subcutaneous injection, e.g. as an injectable solution or suspension, rectal as suppositories, by inhalation or insufflation, e.g. as a powder formulation, as microcrystals or as a spray (e.g. liquid aerosol), transdermal, for example via an transdermal delivery system (TDS) such as a plaster containing the active ingredient or intranasal. For the production of such tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatine, malt, silica gel, starch or derivatives thereof, talc, stearinic acid or their salts, dried skim milk, and the like. For the production of soft capsules one may use excipients as are e.g. vegetable, petroleum, animal or synthetic oils, wax, fat, polyols. For the production of liquid solutions, emulsions or suspensions or syrups one may use as excipients e.g. water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories one may use excipients as are e.g. vegetable, petroleum, animal or synthetic oils, wax, fat and polyols. For aerosol formulations one may use compressed gases suitable for this purpose, as are e.g. oxygen, nitrogen and carbon dioxide. The pharmaceutically useful agents may also contain additives for conservation, stabilization, e.g. UV stabilizers, emulsifiers, sweetener, aromatizers, salts to change the osmotic pressure, buffers, coating additives and antioxidants.

In general, in the case of oral or parenteral administration to adult humans weighing approximately 80 kg, a daily dosage of about 1 mg to about 10,000 mg, preferably from about 5 mg to about 1,000 mg, should be appropriate, although the upper limit may be exceeded when indicated. The daily dosage can be administered as a single dose or in divided doses, or for parenteral administration, it may be given as continuous infusion or subcutaneous injection.

EXAMPLES

*Streptomyces albus* carrying the xiamycin biosynthesis gene cluster was fermented in a large scale (50 L). Both mycelia and culture filtrate were extracted by ethyl acetate to afford a crude extract. After fractionation on flash chromatography on silica, followed by a Sephadex LH-20 column and final purification by HPLC, various dimers were obtained as pure substances. The NMR spectra of all dimers indicated that they have the xiamycin A backbone, yet according to HRESIMS data their molecular formula is $C_{46}H_{48}N_2O_6$ (**2a, 2b, 3a, 3b,** and **4**) and $C_{46}H_{48}N_2SO_8$ (**5, 6** and **7**), respectively. Consequently, they are dimers and sulfonyl dimers of xiamycin, respectively. After examination of NMR spectra respecially COSY and HMBC correlations, their structures were established. Figure 2 shows selected COSY and HMBC correlations for bixiamycins and sulfonylbixiamycins.

The *Streptomyces albus* strain has been deposited at the DSMZ (Inhoffenstr. 7B, 38124 Braunschweig, Germany) by the Leibniz Institute for Natural Product Research and Infection Biology e.V. -Hans-Knöll-Institute (HKI) (Beutenbergstr. 11a, 07745 Jena, Germany) on 20.08.2012 with the deposition number of DSM26342.

Reference compounds **2a/2b** were a pair of atropoisomers. $^{13}$C NMR spectra exhibited a highly conserved pattern as xiamycin. $^{1}$H NMR spectrum exhibited twelve aromatic protons. However, there were some differences in the chemical shifts for all protons in the $^{1}$H NMR spectrum. Based on the HRESIMS data, an only possibility is that **2a/2b** were *N-N* coupled isomers. COSY and HMBC established the structure for the halve of the dimer.

Compounds **3a/3b** were another pair of atropoisomers. Their $^{1}$H NMR spectra showed two sets of signals, indicating that the two halves of the molecular were not identical. Only eleven aromatic protons were visible in the $^{1}$H NMR spectrum. As only a signal for H-21 was visible whereas the counterpart of H-21' was missing in the $^{1}$H NMR spectrum, a *N-C* coupling between N-1 and C-21' was proposed. COSY and HMBC established the structure for the two halves of the dimers.

Compound **4** was another C-N dimer. The $^{1}$H NMR spectra showed two sets of signals, indicating that the two halves of the molecular were not identical. Eleven aromatic signals were visible. COSY and HMBC spectra established the structures for both halves of the isomer. A *N-C* coupled isomer between N-2' and C-6 was proposed and fully established.

HRESIMS of Compound **5, 6** and **7** indicated the presence of a sulfonyl group. Compared to the above mentioned bixiamycins, there were dramatic changes in the UV spectra for **5, 6** and **7** compared to other bixiamycins, a substitution of sulfonyl group in the aromatic chromophore was proposed. $^{1}$H NMR spectrum of compound **5** exhibited eleven aomatic protons. Further analysis of COSY spectrum indicated a substitution at C-6. Thus, a sulfonyl group was located between N-2' and C-6. IR spectrum exhibited the characteristic absorption for sulfonyl group at 1244 and 1167 cm$^{-1}$, respectively.

Both $^{1}$H NMR and $^{13}$C NMR spectra from compound **6** exhibited a similar pattern as xiamycin. However, only six aromatic protons ($\delta$ 8.82, 7.97, 7.93, 7.53, 7.05) were observed. Based on the coupling constant and COSY spectra, the connection between H-5 ($\delta$ 8.82, d, 1.5 Hz), H-7 ($\delta$ 7.97, dd, 8.6, 1.8 Hz) and H-8 ($\delta$ 7.53, d, 8.6 Hz) were confirmed.

Two other singlets ($\delta$ 7.93, 7.05) were from the carbazole ring H-10 and H-21, respectively. Based on the HRESIMS, a sulfonyl group between C(6)-C(6') was proposed. HMBC correlations fully confirmed the structure. IR spectrum exhibited the characteristic absorption for sulfonyl group at 1286 and 1126 $cm^{-1}$, respectively.

Compound 7 represents another sulfonyl bridged dimer of xiamycin as only ten aromatic protons were observed. Based on COSY and $^1H$ NMR spectra, a sulfonyl function was located between C-6 and H-21' and confirmed by HMBC correlations. IR spectrum exhibited the characteristic absorption for sulfonyl group at 1286 and 1121 $cm^{-1}$, respectively.

**General Experimental Procedures.** NMR spectra were recorded on a Bruker Avance III 500 MHz or Avance III 600 MHz spectrometers. IR spectra were recorded on a Bruker FT-IR (IFS 55) spectrometer. UV spectra were recorded on a Cary 1 Bio UV-visible spectrophotometer (Variant). Optical rotation was recorded on a Propol digital automatic polarimeter (Dr. Wolfgang Kernchen GmbH, Seelze, Germany). CD spectra were recorded on a J-810-150s spectropolarimeter (JASCO, Groß Umstadt, Germany). ESIMS data were obtained on a triple quadrupole mass spectrometer (Quattro; VG Biotech, Cheshire, UK). HRESIMS were recorded on a Finnigan TSQ Quantum Ultra AM Thermo Electron. Open column chromatography was performed on silica gel 60 (Merck, 0.04-0.063 mm, 230-400 mesh ASTM) and Sephadex LH-20 (Pharmacia). TLC analysis was performed on silica gel plates (Sil G/UV254, 0.20 mm, Macherey-Nagel). Preparative HPLC was performed on a Waters HPLC system using a Nucleosil 100-5 $C_{18}$ column (5 $\mu$m, 250 x 16 mm) with a UV detector.

**Fermentation and Isolation.** *S. albus* carrying the xia gene cluster was fermented in a 50 L scale fermentor for five days. The fermentation broth was separated into culture filtrate and mycelia by centrifugation. Both were extracted by ethyl acetate, evaporated into dryness and combined into a crude extract. Separation of the crude extract by flash silica gel chromatography (column 50 $\times$ 3 cm, $CH_2Cl_2$-$CH_3OH$ (0-100%) gradient, yielding ten fractions 1-10. Fraction 8 was fractionated by HPLC (RP-$C_{18}$, MeOH-$H_2O$ as gradient) to yield four dimers: **2a** (10 mg), **2b** (5 mg), **3a** (3 mg) and **3b** (1.0 mg). Fraction 9 was fractionated by HPLC (RP-$C_{18}$, MeOH-$H_2O$ as gradient) to yield three dimers: **4** (23.1 mg), **5** (0.5 mg) and **6** (0.8 mg). Fraction 10 containing **7** (1.5 mg) was purified to afford a pure substance.

**Antimicrobial assays.**

MIC assays were carried out to test all dimers and xiamycin against five bacterial test strains, *Bacillus subtilis* ATCC 6633, *Staphylococcus aureus* SG511, *Mycobacterium vaccae* IMET 10670, *Staphylocococus aureus* 134/94, and *Enterococcus faecalis* 1528 R10. The assay was carried out by the standardized broth microdilution method (Clinical and Laboratory Standards Institute, 2006). Results are shown in **Table 1.**

**Table 1.** Antimicrobial activity of xiamcin, bixiamycins and sulfonylbixiamycins (MIC data, in $\mu$g $mL^{-1}$).

*B. sub.: Bacillus subtilis* ATCC 6633; *S. aur.: Staphylococus aureus* SG511; MRSA: *Staphylococus aureus* 134/94; VRE: vancomycin-resistant *Enterococcus faecalis* 1528 R10; *M. vac.: Mycobacterium vaccae* IMET 10670; Cip: ciprofloxacin.

| Compound | Test strain (MIC) | | | | |
|---|---|---|---|---|---|
| | *B. sub* | *S. aur* | MRSA | VRE | *M. vac* |
| **1** | >100 | 50 | >100 | >100 | 50 |
| **2a** | 12.5 | 0.4 | 0.2 | 3.12 | 25 |
| **2b** | 3.12 | 1.56 | 3.12 | 3.12 | 25 |
| **3a** | 50 | 25 | 50 | 50 | 25 |
| **3b** | 25 | 12.5 | 25 | 50 | 25 |
| **4** | 3.12 | 1.56 | 1.56 | 12.5 | 12.5 |
| **5** | 6.25 | 3.12 | 6.25 | 50 | 25 |
| **6** | >100 | 100 | 100 | >100 | 25 |
| **7** | 100 | 25 | 50 | 50 | 25 |
| Cip | <0.05 | 0.1 | 12.5 | 0.78 | 0.1 |
| MeOH | >100 | >100 | >100 | >100 | >100 |

**Antiproliferative and cytotoxic assays.** All compounds were assayed against L-929 mouse fibroblasts (DSM ACC 2) and K562 human chronic myeloid leukemia cells (DSM ACC 10) for their antiproliferative effects ($GI_{50}$), and

against HeLa human cervix carcinoma cells (DSM ACC 57) for their cytotoxic ($CC_{50}$) effects. Inhibitory concentrations are provided as 50% inhibition of cell growth ($GI_{50}$; the concentration needed to reduce the growth of treated cells to half that of untreated cells) or 50% cytotoxic concentration ($CC_{50}$; the concentration that kills 50% of treated cells). No activity was observed for all compounds ($GI_{50}$ and $CC_{50}$ >30 $\mu$g/mL).

**1:** pale yellow crystal; $[\alpha]_D^{21}$ 137.6 (5.3 mg/mL, MeOH); UV/vis: $\lambda_{max}$ nm (log $\varepsilon$): 300 (4.02), 261 (4.05), 238 (4.34), 215 (4.23); CD (0.73 mg/mL, MeOH): $\Delta\varepsilon_{206}$ -9.56, $\Delta\varepsilon_{218}$ 8.47, $\Delta\varepsilon_{240}$ 21.74, $\Delta\varepsilon_{263}$ 8.17, $\Delta\varepsilon_{301}$ 4.83; IR(film): $v_{max}$: 3402.3, 3244.8, 2937.2, 1703.4, 1648.1, 1612.4, 1560.6, 1491.9, 1466.9, 1442.8, 1378.4, 1320.2, 1244.7, 1170.8, 1066.5, 1032.7, 1016.8, 940.8, 877.3, 767.8, 746.3, 717.7, 673.6, 666.5, 661.3, 652.2, 640.5 $cm^{-1}$; ESIMS: $[M - H]^-$ = 362.2, $[2M - H]^-$ = 724.8, $[M + H]^+$ = 364.1, $[M + NH_4]^+$ = 380.8, $[2M + NH_4]^+$ = 743.9, $[2M + Na]^+$ = 749.0, HRESIMS: found $[M - H]^-$ 362.177 (calculated 362.1757 for $C_{23}H_{24}NO_3$).

**2a:** pale yellow solid; $[\alpha]_D^{23}$ 42.8 (6.67 mg/mL, MeOH); UV/vis: $\lambda_{max}$ nm (log $\varepsilon$): 333 (3.73), 319 (3.84), 296 (4.46), 250 (4.48), 233 (4.80), 217 (4.56); CD (0.11 mg/mL, MeOH): $\Delta\varepsilon_{203}$ - 4.00, $\Delta\varepsilon_{210}$ 3.90, $\Delta\varepsilon_{219}$ 3.58, $\Delta\varepsilon_{236}$ 10.46, $\Delta\varepsilon_{250}$ 2.92, $\Delta\varepsilon_{260}$ 2.87; IR (film) $v_{max}$ 3399.9, 2935.1, 2875.3, 1703.8, 1627.6, 1609.3, 1468.5, 1455.9, 1232.3, 1067.4, 1019.2, 997.9 $cm^{-1}$; ESIMS $[M - H]^-$ 723.3, $[M + H]^+$ 725.4; HRESIMS m/z 725.3602 $[M + H]^+$ (calcd. for $C_{46}H_{49}N_2O_6$ 725.3585); NMR data, see **Table 2.**

**2b:** pale yellow solid; $[\alpha]_D^{23}$ 82.9 (3.3 mg/mL, MeOH); UV/vis: $\lambda_{max}$ nm (log $\varepsilon$): 332 (3.90), 319 (4.02), 296 (4.52), 250 (4.56), 234 (4.80), 217 (4.90); CD (0.11 mg/mL, MeOH): $\Delta\varepsilon_{207}$ - 9.67, $\Delta\varepsilon_{219}$ 9.00, $\Delta\varepsilon_{240}$ 19.73, $\Delta\varepsilon_{253}$ 8.57, $\Delta\varepsilon_{298}$ 11.65; IR (film) $v_{max}$ 3337.2, 2944.7, 2833.9, 1652.7, 1449.2, 1416.5, 1115.6, 1076.1, 1020.2 $cm^{-1}$; ESIMS $[M - H]^-$ 723.3, $[M + H]^+$ 725.4, $[M + NH_4]^+$ 742.4, $[M + Na]^+$ 747.3, NMR data, see **Table 2.**

**Table 2.** NMR data for compounds **2a** and **2b** (MeOD).

| Position | $^1$H NMR $\delta$ (*J* in Hz) (600 MHz) **2a** | $^1$H NMR $\delta$ (*J* in Hz) (600 MHz) **2b** | $^{13}$C NMR $\delta$ (150 MHz) **2a** | $^{11}$C NMR $\delta$ (125 MHz) **2b** |
|---|---|---|---|---|
| 1/1’ | - | - | - | - |
| 2/2’ | - | - | 139.7 | 139.7 |
| 3/3’ | - | - | 122.1 | 121.4 |
| 4/4’ | - | - | 123.5 | 123.6 |
| 5/5’ | 8.01 (d, 7.7) | 8.17 (d, 7.7) | 121.8 | 121.8 |
| 6/6’ | 7.14 (t, 6.8) | 7.27 (t, 6.8) | 121.3 | 122.1 |
| 7/7’ | 7.12 (t, 6.8) | 7.25 (t, 6.8) | 127.2 | 127.2 |
| 8/8’ | 6.57 (d, 7.0) | 6.73 (d, 6.8) | 109.6 | 109.7 |
| 9/9’ | - | - | 144.4 | 144.5 |
| 10/10’ | 8.05 (s) | 8.16 (s) | 117.4 | 117.6 |
| 11/11’ | - | - | 141.4 | 141.6 |
| 12/12’ | - | - | 38.4 | 38.5 |
| 13/13’ | 2.54 (m); 1.71 (m) | 2.69 (td, 13.1, 3.0); 1.80 (m) | 38.8 | 38.9 |
| 14/14’ | 1.83 (m) | 1.93 (m) | 28.5 | 28.6 |
| 15/15’ | 4.04 (dd, 8.1, 8.1) | 4.09 (dd, 8.6, 7.1) | 76.2 | 76.2 |
| 16/16’ | - | - | 54.7 | 54.8 |
| 17/17’ | 2.02 (dd, 12.5, 2.2) | 2.13 (dd, 12.5, 2.0) | 47.5 | 47.7 |
| 18/18’ | 1.72 (m); 1.30 (m) | 1.92 (m); 1.45 (m) | 22.2 | 22.3 |
| 19/19’ | 2.56 (m); 2.48 (m) | 2.86 (m); 2.83 (m) | 31.6 | 31.8 |
| 20/20’ | - | - | 135.7 | 135.8 |
| 21/21’ | 6.32 (s) | 6.45 (s) | 109.1 | 109.1 |
| 22/22’ | 1.16 (s) | 1.33 (s) | 26.3 | 26.2 |
| 23/23’ | 1.13 (s) | 1.22 (s) | 11.3 | 11.4 |
| 24/24’ | - | - | 181.0 | 181.2 |

**3a:** pale yellow solid; $[\alpha]_D^{23}$ 149.4 (0.55 mg/mL, MeOH); UV/vis: $\lambda_{max}$ nm (log $\varepsilon$): 343 (3.83), 329 (3.87), 300 (4.38), 261 (4.39), 239 (4.69); CD (0.33 mg/mL, MeOH): $\Delta\varepsilon_{266}$ -29.07, $\Delta\varepsilon_{220}$ 12.27, $\Delta\varepsilon_{239}$ 24.50, $\Delta\varepsilon_{264}$ 16.72, $\Delta\varepsilon_{303}$ 22.67, $\Delta\varepsilon_{346}$ -5.97; IR (film) $v_{max}$ 2930.3, 1715.4, 1698.0, 1457.0, 1236.2, 1063.6, 1018.2, 744.4 $cm^{-1}$; ESIMS $[M - H]^-$ 723.3, NMR data, see **Table 3.**

**Table 3.** NMR data for **3a** (MeOD).

| Position | $^1H$ NMR $\delta$ ($J$ in Hz) (600 MHz) part A | $^1H$ NMR $\delta$ ($J$ in Hz) (600 MHz) part B | $^{13}C$ NMR $\delta$ (125 MHz) part A | $^{13}C$ NMR $\delta$ (125 MHz) part B |
|---|---|---|---|---|
| 1/1’ | - | - | - | - |
| 2/2’ | - | - | 138.0 | 140.5 |
| 3/3’ | - | - | 124.7 | 123.4 |
| 4/4’ | - | - | 124.7 | 125.0 |
| 5/5’ | 8.05 (d, 7.8) | 8.15 (d, 7.7) | 120.8 | 121.1 |
| 6/6’ | 7.13 (t, 6.8) | 7.19 (t, 7.0) | 119.9 | 120.4 |
| 7/7’ | 7.26 (t, 6.8) | 7.27 (t, 6.8) | 126.6 | 126.6 |
| 8/8’ | 7.19 (d, 8.0) | 6.79 (d, 8.0) | 112.1 | 110.5 |
| 9/9’ | - | - | 142.1 | 142.2 |
| 10/10’ | 8.23 (s) | 8.12 (s) | 117.4 | 117.0 |
| 11/11’ | - | - | 143.0 | 143.1 |
| 12/12’ | - | - | 39.1 | 39.2 |
| 13/13’ | 2.69 (m); 1.77 (m) | 2.74 (m); 1.95 (m) | 38.4 | 38.6 |
| 14/14’ | 1.83 (m) | 1.83 (m) | 28.6 | 28.7 |
| 15/15’ | 4.07 (dd, 9.1, 7.3) | 4.11 (dd, 9.1, 7.3) | 76.3 | 76.2 |
| 16/16’ | - | - | 54.8 | 54.9 |
| 17/17’ | 2.12 (dd, 12.5, 2.1) | 2.16 (dd, 12.5, 1.9) | 47.6 | 47.9 |
| 18/18’ | 1.85 (m); 1.30 (m) | 1.98 (m); 1.46 (m) | 21.8 | 22.4 |
| 19/19’ | 2.42 (m); 2.37 (m) | 2.94 (dd, 167, 6.4); 2.86 (m) | 26.9 | 31.9 |
| 20/20’ | - | - | 133.2 | 135.0 |
| 21/21’ | - | 6.53 (s) | 118.7 | 109.7 |
| 22/22’ | 1.39 (s) | 1.33 (s) | 26.3 | 26.2 |
| 23/23’ | 1.23 (s) | 1.19 (s) | 11.3 | 11.4 |
| 24/24’ | - | - | 181.2 | 181.2 |

**3b:** pale yellow solid; $[\alpha]_D^{23}$ 25.2 (0.55 mg/mL, MeOH); UV/vis: $\lambda_{max}$ nm (log $\varepsilon$): 343 (3.83), 329 (3.85), 300 (4.38), 261 (4.38), 239 (4.68), 218 (4.51); CD (0.73 mg/mL, MeOH): $\Delta\varepsilon_{212}$ 7.48, $\Delta\varepsilon_{220}$ -1.33, $\Delta\varepsilon_{237}$ 18.21, $\Delta\varepsilon_{252}$ 4.86, $\Delta\varepsilon_{264}$ -5.32, $\Delta\varepsilon_{288}$ 1.42, $\Delta\varepsilon_{304}$ -6.56; IR (film) $v_{max}$ 2925.5, 2853.2, 1464.7, 1068.4, 1018.2, 999.0, 743.4 $cm^{-1}$; ESIMS $[M - H]^-$ 723.5, NMR data, see **Table 4.**

**Table 4.** NMR data for **3b** (MeOD).

| Position | $^1H$ NMR $\delta$ ($J$ in Hz) (500 MHz) part A | $^1H$ NMR $\delta$ ($J$ in Hz) (500 MHz) part B | $^{13}C$ NMR $\delta$ (150 MHz) part A | $^{13}C$ NMR $\delta$ (150 MHz) part B |
|---|---|---|---|---|
| 1/1’ | - | - | - | - |
| 2/2’ | - | - | 138.0 | 140.4 |
| 3/3’ | - | - | 124.7 | 123.3 |
| 4/4’ | - | - | 124.7 | 125.0 |
| 5/5’ | 8.09 (d, 7.8) | 8.15 (d, 7.8) | 120.8 | 121.0 |
| 6/6’ | 7.13 (t, 7.9) | 7.20 (t, 7.0) | 119.9 | 120.5 |
| 7/7’ | 7.26 (t, 6.8) | 7.27 (t, 6.8) | 126.6 | 126.7 |
| 8/8’ | 7.20 (d, 7.7) | 6.80 (d, 8.1) | 112.0 | 110.3 |

(continued)

| Position | $^{1}H$ NMR $\delta$ ($J$ in Hz) (500 MHz) part A | $^{1}H$ NMR $\delta$ ($J$ in Hz) (500 MHz) part B | $^{13}C$ NMR $\delta$ (150 MHz) part A | $^{13}C$ NMR $\delta$ (150 MHz) part B |
|---|---|---|---|---|
| 9/9' | - | - | 142.1 | 142.3 |
| 10/10' | 8.23 (s) | 8.12 (s) | 117.5 | 117.1 |
| 11/11' | - | - | 143.2 | 142.9 |
| 12/12' | - | - | 38.6 | 38.4 |
| 13/13' | 2.76 (m); 1.95 (m) | 2.69 (m); 1.85 (m) | 39.0 | 39.2 |
| 14/14' | 1.93 (m) | 1.93 (m) | 28.6 | 28.6 |
| 15/15' | 4.11 (dd, 9.1, 7.5) | 4.11 (dd, 9.1, 7.5) | 76.3 | 76.2 |
| 16/16' | - | - | 54.9 | 54.8 |
| 17/17' | 2.16 (d, 12.5) | 2.16 (d, 12.5) | 47.5 | 47.8 |
| 18/18' | 1.83 (m); 1.27 (m) | 1.93 (m); 1.48 (m) | 22.5 | 21.8 |
| 19/19' | 2.37 (m) | 2.92 (m) | 26.7 | 32.0 |
| 20/20' | - | - | 133.3 | 135.0 |
| 21/21' | - | 6.51 (s) | 118.7 | 109.7 |
| 22/22' | 1.40 (s) | 1.31 (s) | 26.4 | 26.3 |
| 23/23' | 1.22 (s) | 1.19 (s) | 11.4 | 11.4 |
| 24/24' | - | - | 181.5 | 181.5 |

**4:** pale yellow solid; $[\alpha]_D^{25}$ 133.9 (1.63 mg/mL, MeOH); UV/vis: $\lambda_{max}$ nm (log $\varepsilon$): 333 (3.78), 301 (4.33), 265(4.39), 240 (4.69), 219 (4.42); CD (0.38 mg/mL, MeOH): $\Delta\varepsilon_{206}$ -9.70, $\Delta\varepsilon_{225}$ 9.64, $\Delta\varepsilon_{242}$ 19.74, $\Delta\varepsilon_{266}$ 8.41, $\Delta\varepsilon_{303}$ 4.68, $\Delta\varepsilon_{353}$ -2.86; IR (film) $v_{max}$ 2938.0, 2833.8, 1697.1, 1494.6, 1468.5, 1235.2, 1018.2, 745.4 $cm^{-1}$; ESIMS $[M - H]^-$ 723.5, NMR data, see **Table 5.**

**Table 5.** NMR data for **4** (MeOD).

| Position | $^{1}H$ NMR $\delta$ ($J$ in Hz) (600 MHz) part A | $^{1}H$ NMR $\delta$ ($J$ in Hz) (600 MHz) part B | $^{13}C$ NMR $\delta$ (125 MHz) part A | $^{13}C$ NMR $\delta$ (125 MHz) part B |
|---|---|---|---|---|
| 1/1' | - | - | - | - |
| 2/2' | - | - | 140.9 | 142.0 |
| 3/3' | - | - | 122.7 | 122.9 |
| 4/4' | - | - | 125.4 | 124.5 |
| 5/5' | 7.97 (d, 2.0) | 7.99 (d, 8.2) | 120.2 | 120.7 |
| 6/6' | - | 7.08 (t, 7.8) | 129.8 | 120.2 |
| 7/7' | 7.21 (dd, 8.3, 2.0) | 7.16 (t, 7.3) | 125.6 | 126.4 |
| 8/8' | 7.39 (d, 8.4) | 7.11 (d, 8.0) | 112.4 | 110.5 |
| 9/9' | - | - | 140.7 | 143.7 |
| 10/10' | 7.81 (s) | 7.96 (s) | 116.7 | 116.5 |
| 11/11' | - | - | 142.2 | 142.5 |
| 12/12' | - | - | 38.2 | 38.3 |
| 13/13' | 2.29 (d, 11.4); 1.45 (m) | 2.56 (d, 13.1); 1.65 (m) | 38.7 | 38.9 |
| 14/14' | 1.62 (m) | 1.83 (m) | 28.6 | 28.4 |
| 15/15' | 3.96 (dd, 10.9, 4.8) | 4.05 (dd, 8.2, 7.9) | 76.2 | 76.2 |
| 16/16' | - | - | 54.7 | 54.8 |
| 17/17' | 2.06 (t, 14.2) | 2.06 (t, 14.2) | 47.6 | 47.7 |
| 18/18' | 1.95 (m); 1.52 (m) | 1.77 (m); 1.34(m) | 22.4 | 22.4 |
| 19/19' | 2.98 (m) | 2.74 (m) | 31.9 | 31.9 |
| 20/20' | - | - | 134.9 | 134.5 |
| 21/21' | 6.99 (s) | 6.85 (s) | 111.2 | 109.9 |
| 22/22' | 1.08 (s) | 1.18 (s) | 26.2 | 26.3 |
| 23/23' | 1.17 (s) | 1.14 (s) | 11.3 | 11.4 |

(continued)

| Position | $^1$H NMR $\delta$ ($J$ in Hz) (600 MHz) part A | $^1$H NMR $\delta$ ($J$ in Hz) (600 MHz) part B | $^{13}$C NMR $\delta$ (125 MHz) part A | $^{13}$C NMR $\delta$ (125 MHz) part B |
|---|---|---|---|---|
| 24/24' | - | - | 181.2 | 181.3 |

**5:** pale yellow solid; $[\alpha]_D^{25}$ 134.1 (0.50 mg/mL, MeOH); UV/vis: $\lambda_{max}$ nm (log $\varepsilon$): 285 (4.46), 224 (4.58); CD (0.33 mg/mL, MeOH): $\Delta\varepsilon_{230}$ 19.12, $\Delta\varepsilon_{290}$ 9.50; IR (film) $v_{max}$ 2927.4, 2853.2, 1652.7, 1243.9, 1166.7, 1136.8, 1016.3, 937.2 $cm^{-1}$; HRESIMS m/z 789.3217 $[M + H]^+$, (calcd. for $C_{46}H_{49}N_2SO_8$ 789.3204); NMR data, see **Table 6.**

**Table 6.** NMR data for **5** (MeOD).

| Position | $^1$H NMR $\delta$ ($J$ in Hz) (500 MHz) part A | $^1$H NMR $\delta$ ($J$ in Hz) (500 MHz) part B | $^{13}$C NMR $\delta$ (150 MHz) part A | $^{13}$C NMR $\delta$ (150 MHz) part B |
|---|---|---|---|---|
| 1/1' | - | - | - | - |
| 2/2' | - | - | 140.7 | 138.3 |
| 3/3' | - | - | 122.2 | 126.2 |
| 4/4' | - | - | 124.1 | 128.2 |
| 5/5' | 8.56 (s) | 7.89 (d, 8.6) | 120.5 | 120.8 |
| 6/6' | - | 7.29 (t, 7.4) | 127.8 | 124.9 |
| 7/7' | 7.65 (d, 8.8) | 7.45 (t, 7.4) | 124.1 | 127.8 |
| 8/8' | 7.89 (d, 7.6) | 8.35 (d, 8.4) | 111.7 | 116.3 |
| 9/9' | - | - | 144.7 | 140.1 |
| 10/10' | 7.82 (s) | 7.82 (s) | 116.8 | 116.9 |
| 11/11' | - | - | 143.6 | 146.9 |
| 12/12' | - | - | 38.3 | 38.3 |
| 13/13' | 2.49 (m); 1.63 (m) | 2.32 (m); 1.53 (m) | 38.4 | 38.4 |
| 14/14' | 1.82 (m) | 1.75 (m) | 28.4 | 28.4 |
| 15/15' | 4.03 (m) | 4.03 (m) | 76.2 | 76.2 |
| 16/16' | - | - | 54.8 | 54.8 |
| 17/17' | 2.08 (m) | 2.08 (m) | 47.5 | 47.4 |
| 18/18' | 1.92 (m); 1.53 (m) | 1.92 (m); 1.53 (m) | 22.4 | 22.4 |
| 19/19' | 2.85 (m) | 3.15 (m); 3.05 (m) | 31.9 | 32.3 |
| 20/20' | - | - | 136.2 | 136.3 |
| 21/21' | 7.03 (s) | 8.08 (s) | 111.5 | 116.0 |
| 22/22' | 1.14 (s) | 1.14 (s) | 26.0 | 26.0 |
| 23/23' | 1.19 (s) | 1.19 (s) | 11.5 | 11.5 |
| 24/24' | - | - | 181.7 | 181.7 |

**6:** pale yellow solid; $[\alpha]_D^{23}$ 65.2 (0.90 mg/mL, MeOH); UV/vis: $\lambda_{max}$ nm (log $\varepsilon$): 293 (4.38), 272 (4.31), 220 (4.37); CD (0.68 mg/mL, MeOH): $\Delta\varepsilon_{236}$ 18.95, $\Delta\varepsilon_{275}$ 8.25; IR (film) $v_{max}$ 3335.3, 2931.3, 1602.6, 1286.3, 1242.9, 1141.7, 1126.2, 875.5, 804.2 $cm^{-1}$; ESIMS $[M - H]^-$ 787.3, NMR data, see **Table 7.**

**Table 7.** NMR data for **6** (MeOD).

| Position | $^1$H NMR $\delta$ ($J$ in Hz) (600 MHz) | $^{13}$C NMR $\delta$ (150 MHz) |
|---|---|---|
| 1/1' | - | - |
| 2/2' | - | 140.8 |
| 3/3' | - | 122.5 |
| 4/4' | - | 124.8 |
| 5/5' | 8.82 (d, 1.5) | 120.8 |
| 6/6' | - | 133.5 |

(continued)

| Position | $^1H$ NMR $\delta$ (J in Hz) (600 MHz) | $^{13}C$ NMR $\delta$ (150 MHz) |
|---|---|---|
| 7/7' | 7.97 (dd, 8.6, 1.8) | 125.2 |
| 8/8' | 7.47 (d, 8.6) | 112.0 |
| 9/9' | - | 144.2 |
| 10/10' | 7.93 (s) | 117.1 |
| 11/11' | - | 143.4 |
| 12/12' | - | 38.3 |
| 13/13' | 2.36 (m); 1.54 (m) | 38.6 |
| 14/14' | 1.82 (m) | 28.5 |
| 15/15' | 4.01 (dd, 11.4, 5.0) | 76.3 |
| 16/16' | - | 54.8 |
| 17/17' | 2.05 (dd, 12.5, 2.0) | 47.6 |
| 18/18' | 1.93 (m); 1.52(m) | 22.4 |
| 19/19' | 3.04 (dd, 16.7, 6.6); 2.96 (m) | 31.9 |
| 20/20' | - | 135.7 |
| 21/21' | 7.05 (s) | 111.4 |
| 22/22' | 1.07 (s) | 26.0 |
| 23/23' | 1.19 (s) | 11.4 |
| 24/24' | - | 181.6 |

**7:** pale yellow solid; $[\alpha]_D^{23}$ 68.0 (0.80 mg/mL, MeOH); UV/vis: $\lambda_{max}$ nm (log $\varepsilon$): 360 (3.94), 346 (3.95), 290 (4.58), 222 (4.76); CD (0.50 mg/mL, MeOH): $\Delta\varepsilon_{217}$ 6.73, $\Delta\varepsilon_{238}$ 18.21, $\Delta\varepsilon_{271}$ 9.97, $\Delta\varepsilon_{289}$ -7.12, $\Delta\varepsilon_{311}$ 2.36; IR (film) $v_{max}$ 2931.3, 2840.6, 1652.7, 1467.6, 1286.3, 1241.0, 1121.4, 1015.3 $cm^{-1}$; ESIMS $[M - H]^-$ 787.3, NMR data, see **Table 8.**

**Table 8.** NMR data for **7** (MeOD).

| Position | $^1H$ NMR $\delta$ (J in Hz) (600 MHz) | $^1H$ NMR $\delta$ (J in Hz) (600 MHz) | $^{11}C$ NMR $\delta$ (150 MHz) | $^{11}C$ NMR $\delta$ (150 MHz) |
|---|---|---|---|---|
| | part A | part B | part A | part B |
| 1/1' | - | - | - | - |
| 2/2' | - | - | 140.9 | 138.2 |
| 3/3' | - | - | 122.4 | 125.7 |
| 4/4' | - | - | 124.3 | 123.2 |
| 5/5' | 8.61 (d, 1.7) | 8.12 (d, 8.9) | 120.5 | 120.9 |
| 6/6' | - | 7.24 (dd, 7.8, 7.3) | 132.9 | 120.8 |
| 7/7' | 7.75 (dd, 8.7, 1.7) | 7.45 (t, 7.3) | 124.7 | 127.7 |
| 8/8' | 7.43 (d, 8.7) | 7.65 (d, 8.1) | 111.8 | 112.5 |
| 9/9' | - | - | 144.4 | 141.7 |
| 10/10' | 8.00 (s) | 8.42 (s) | 117.2 | 123.4 |
| 11/11' | - | - | 143.6 | 143.0 |
| 12/12' | - | - | 38.8 | 39.2 |
| 13/13' | 2.57 (m); 1.67 (m) | 2.61 (m); 1.67 (m) | 38.4 | 39.0 |
| 14/14' | 1.84 (m) | 1.84 (m) | 28.5 | 28.5 |
| 15/15' | 4.03 (dd, 8.9, 8.5) | 4.05 (dd, 9.1, 7.3) | 76.3 | 75.9 |
| 16/16' | - | - | 54.7 | 54.6 |
| 17/17' | 2.12 (dd, 11.5, 2.0) | 1.92 (dd, 12.6, 1.5) | 47.7 | 46.6 |
| 18/18' | 2.00 (m); 1.55 (m) | 1.79 (m); 1.45 (m) | 22.4 | 21.8 |
| 19/19' | 3.11 (dd, 6.6, 6.4); 3.03 (m) | 3.49 (dd, 8.2, 7.9); 2.89 (m) | 32.0 | 29.2 |
| 20/20' | - | - | 136.1 | 134.5 |
| 21/21' | 7.13(s) | - | 111.6 | 121.4 |

(continued)

| Position | $^1H$ NMR $\delta$ ($J$ in Hz) (600 MHz) | $^1H$ NMR $\delta$ ($J$ in Hz) (600 MHz) | $^{11}C$ NMR $\delta$ (150 MHz) | $^{11}C$ NMR $\delta$ (150 MHz) |
|---|---|---|---|---|
| | part A | part B | part A | part B |
| 22/22' | 1.25 (s) | 1.24 (s) | 26.0 | 26.2 |
| 23/23' | 1.21 (s) | 1.13 (s) | 11.5 | 11.4 |
| 24/24' | - | - | 181.6 | 181.6 |

**References:**

Takada K, Kajiwara H, Imamura N.Oridamycins A and B, anti-Saprolegnia parasitica indolosesquiterpenes isolated from Streptomyces sp. KS84.J Nat Prod. 2010 Apr 23;73(4):698-701.

Ding L, Münch J, Goerls H, Maier A, Fiebig HH, Lin WH, Hertweck C.Xiamycin, a pentacyclic indolosesquiterpene with selective anti-HIV activity from a bacterial mangrove endophyte.Bioorg Med Chem Lett. 2010 Nov 15;20(22):6685-7. Epub 2010 Sep 9.

Ding L, Maier A, Fiebig HH, Lin WH, Hertweck C.A family of multicyclic indolosesquiterpenes from a bacterial endophyte.Org Biomol Chem. 2011 Jun 7;9(11):4029-31. Epub 2011 Apr 28.

Li H, Zhang Q, Li S, Zhu Y, Zhang G, Zhang H, Tian X, Zhang S, Ju J, Zhang C.Identification and characterization of xiamycin A and oxiamycin gene cluster reveals an oxidative cyclization strategy tailoring indolosesquiterpene biosynthesis.J Am Chem Soc. 2012 May 30;134(21):8996-9005. Epub 2012 May 16.

Clinical and Laboratory Standards Institute. Methods for dilution antimicrobial susceptibility tests for bacteria that grow aerobically; Approved standard M7-A7; PA. 2006.

## Claims

1. A compound of formula (II):

X-A-Y (II)

wherein A is a bond, a sulphur atom or a group of formula -SO- or $-SO_2-$,
wherein X is selected from the following structures

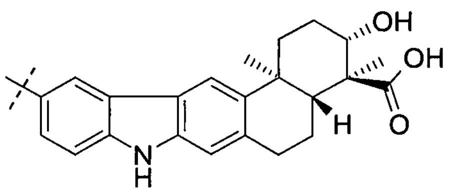

or

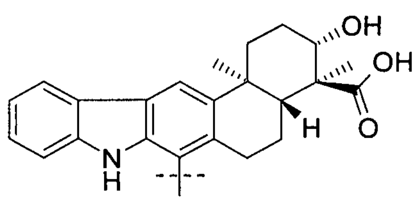

and wherein Y has the following formula (I) wherein one hydrogen atom is replaced by the bond to A or X

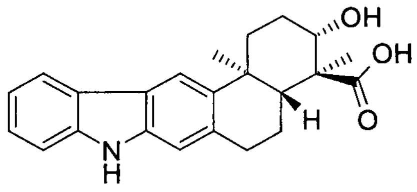

(I)

or a pharmaceutically acceptable salt, solvate or hydrate or a pharmaceutically acceptable formulation thereof.

2. A compound according claim 1, wherein Y is selected from the following structures:

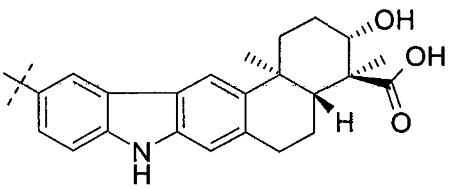

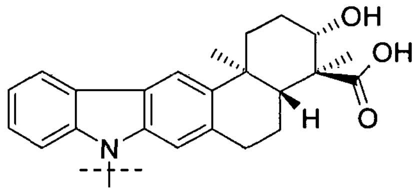

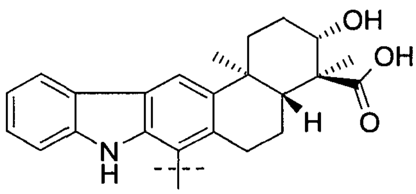

3. A compound according claim 1 or 2, wherein A is a bond.

4. A compound according claim 1 or 2, wherein A is a group of formula $-SO_2-$.

5. A compound according claim 1 or 2, wherein A is a group of formula -SO-.

6. A compound according claim 1 or 2, wherein A is a sulphur atom.

7. A compound according to claim 1, selected from the following compounds:

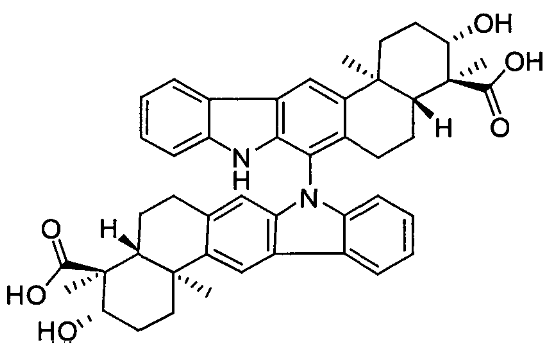

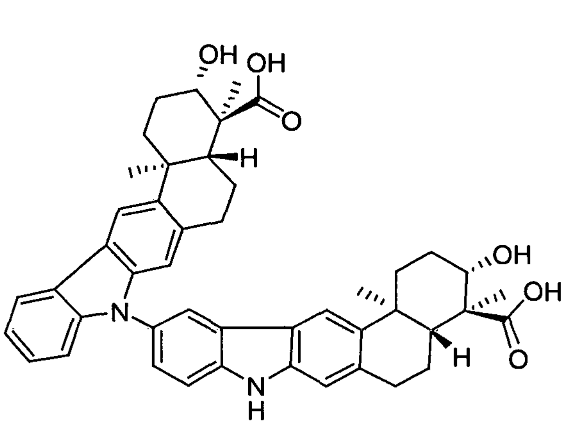

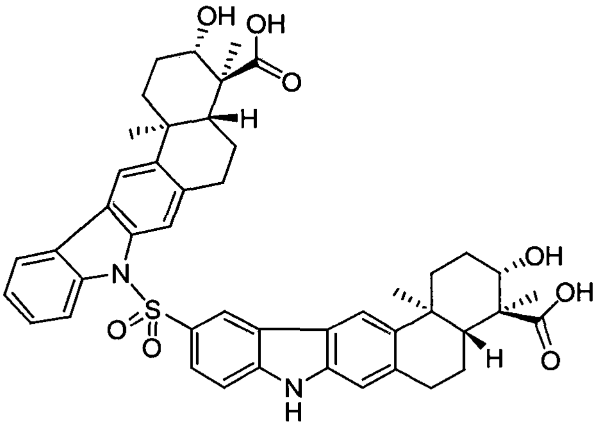

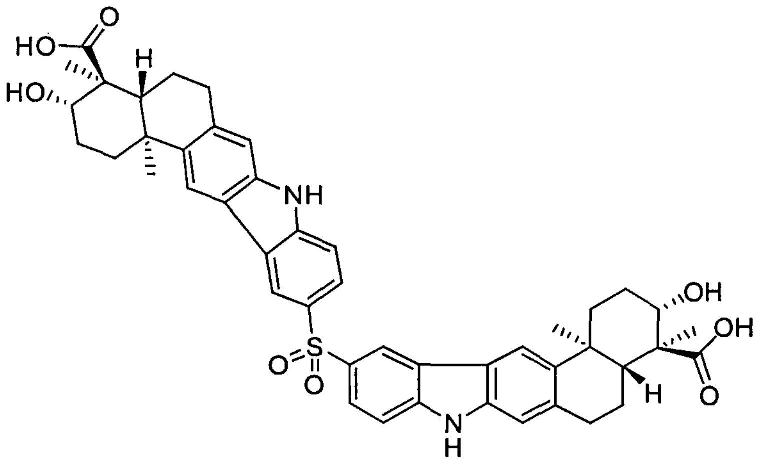

and

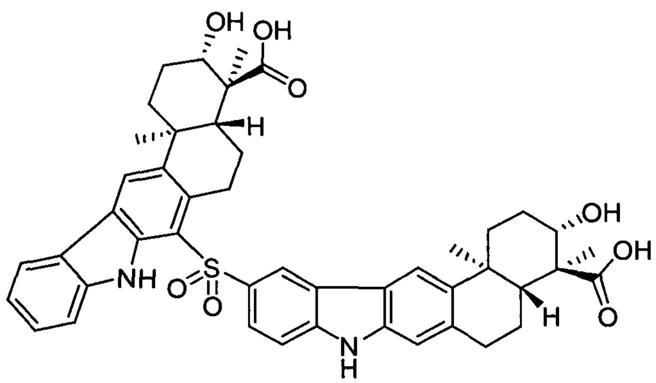

8. A pharmaceutical composition comprising a compound according to anyone of the preceding claims 1 to 7 or a pharmaceutically acceptable salt, solvate or hydrate thereof, optionally in combination with a pharmaceutically acceptable carrier.

9. A compound according to anyone of claims 1 to 7 or a pharmaceutical composition according to claim 8 for use in the treatment of bacterial infections.

10. *Streptomyces albus* strain which has been deposited at the DSMZ (Inhoffenstr. 7B, 38124 Braunschweig, Germany) by the Leibniz Institute for Natural Product Research and Infection Biology e.V. - Hans-Knöll-Institute (HKI) (Beutenbergstr. 11a, 07745 Jena, Germany) on 20.08.2012 with the deposition number of DSM26342.

**Patentansprüche**

**1.** Verbindung der Formel (II):

X-A-Y (II)

wobei A eine Bindung, ein Schwefelatom oder eine Gruppe der Formel -SO- oder $-SO_2-$ ist, wobei X aus den folgenden Strukturen ausgewählt wird

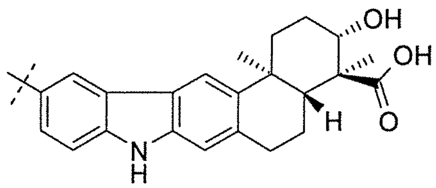

oder

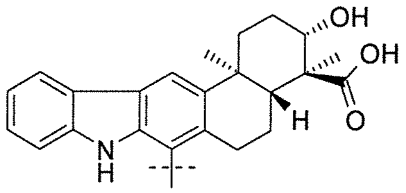

und wobei Y die folgende Formel (I) aufweist, wobei ein Wasserstoffatom durch die Bindung zu A oder X ersetzt ist

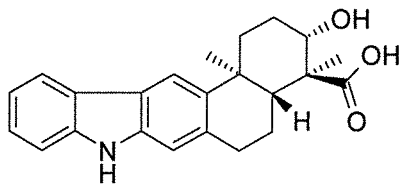

(I)

oder ein pharmazeutisch verträgliches Salz, Solvat oder Hydrat oder eine pharmazeutisch verträgliche Formulierung davon.

**2.** Verbindung nach Anspruch 1, wobei Y aus den folgenden Strukturen ausgewählt wird:

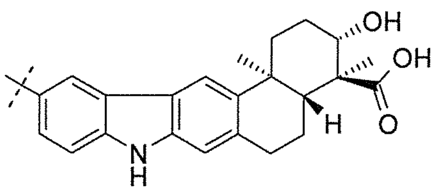

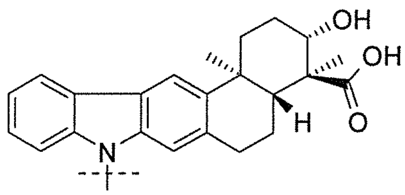

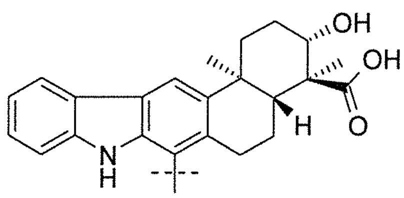

**3.** Verbindung nach Anspruch 1 oder 2, wobei A eine Bindung ist.

**4.** Verbindung nach Anspruch 1 oder 2, wobei A eine Gruppe der Formel $-SO_2-$ ist.

**5.** Verbindung nach Anspruch 1 oder 2, wobei A eine Gruppe der Formel -SO- ist.

**6.** Verbindung nach Anspruch 1 oder 2, wobei A ein Schwefelatom ist.

**7.** Verbindung nach Anspruch 1, die aus den folgenden Verbindungen ausgewählt wird:

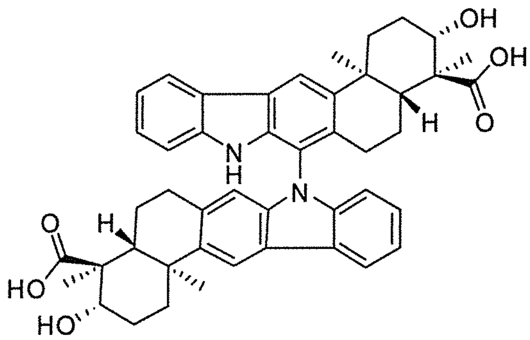

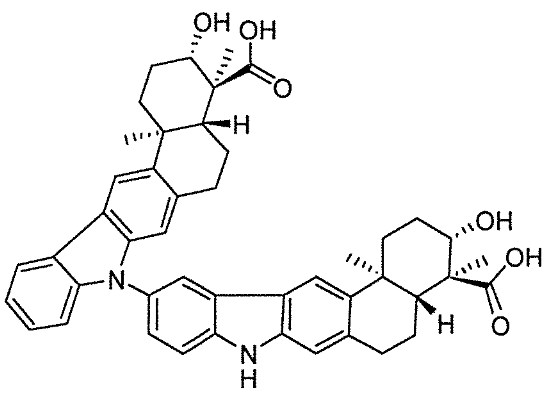

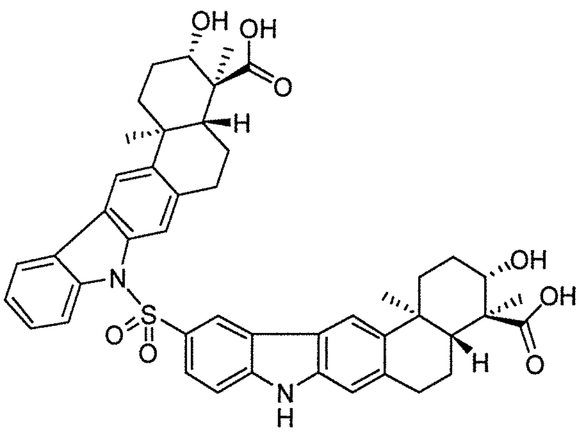

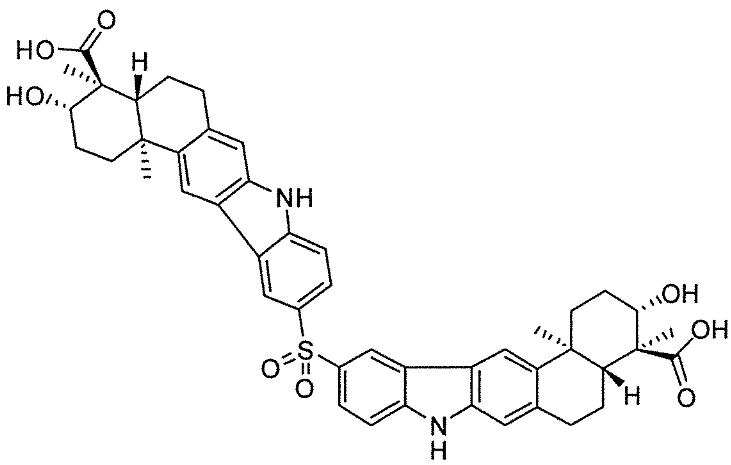

und

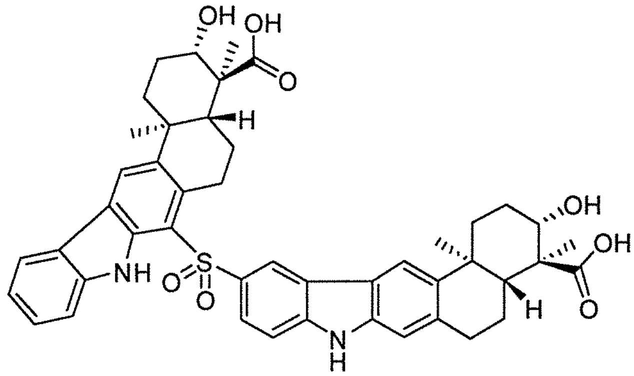

8. Pharmazeutische Zusammensetzung, umfassend eine Verbindung nach einem der Ansprüche 1 bis 7 oder ein pharmazeutisch verträgliches Salz, Solvat oder Hydrat davon, gegebenenfalls in Kombination mit einem pharmazeutisch verträglichen Träger.

9. Verbindung nach einem der Ansprüche 1 bis 7 oder pharmazeutische Zusammensetzung nach Anspruch 8 zur Verwendung bei der Behandlung von bakteriellen Infektionen.

10. *Streptomyces albus* Stamm, der bei der DSMZ (Inhoffenstr. 7B, 38124 Braunschweig, Deutschland) durch das Leibniz-Institut für Naturstoffforschung und Infektionsbiologie e. V. - Hans-Knoll-Institut (HKI) (Beutenbergstr. 11a, 07745 Jena, Deutschland) am 20.08.2012 mit der Hinterlegungsnummer DSM26342 hinterlegt wurde.

## Revendications

1. Composé de formule (II) :

**X-A-Y** **(II)**

où A est une liaison, un atome de soufre ou un groupe de formule -SO- ou $-SO_2-$,
où X est choisi parmi les structures suivantes

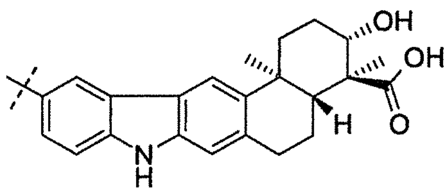

ou

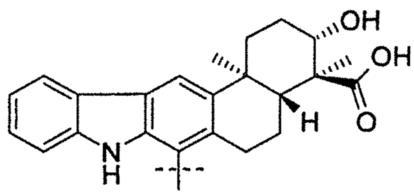

et où Y répond à la formule (I) suivante dans laquelle un atome d'hydrogène est remplacé par la liaison à A ou X

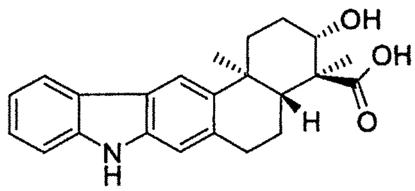

(I)

ou sel pharmaceutiquement acceptable, solvate ou hydrate ou formulation pharmaceutiquement acceptable de celui-ci.

**2.** Composé selon la revendication 1, dans lequel Y est choisi parmi les structures suivantes :

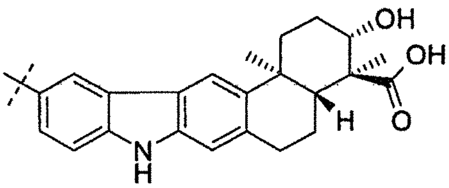

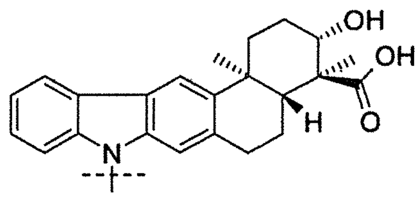

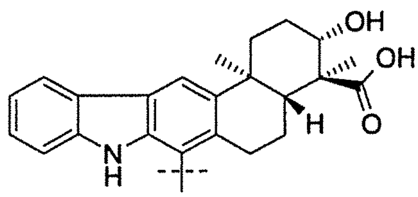

**3.** Composé selon la revendication 1 ou 2, dans lequel A est une liaison.

**4.** Composé selon la revendication 1 ou 2, dans lequel A est un groupe de formule $-SO_2-$.

**5.** Composé selon la revendication 1 ou 2, dans lequel A est un groupe de formule -SO-.

**6.** Composé selon la revendication 1 ou 2, dans lequel A est un atome de soufre.

**7.** Composé selon la revendication 1, choisi parmi les composés suivants :

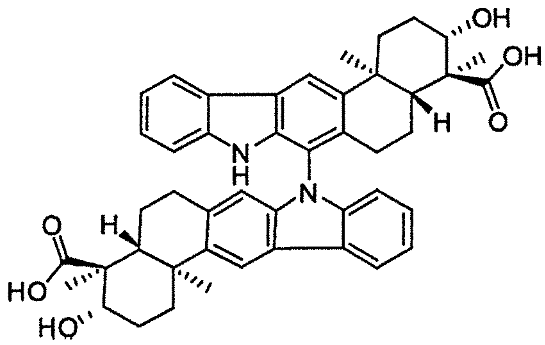

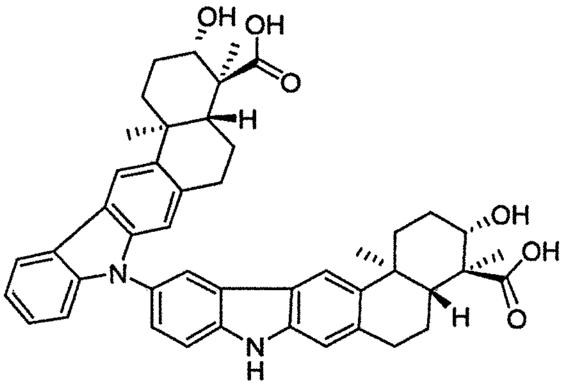

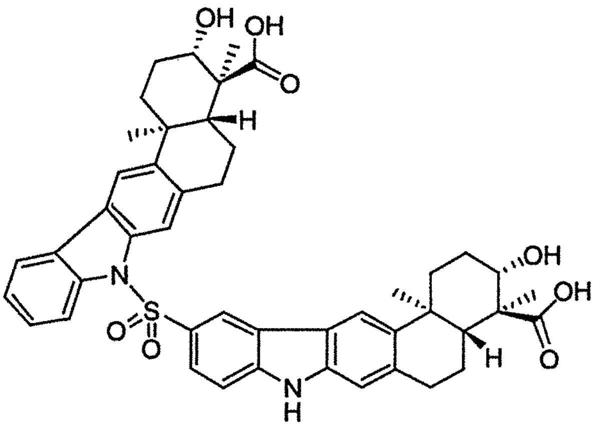

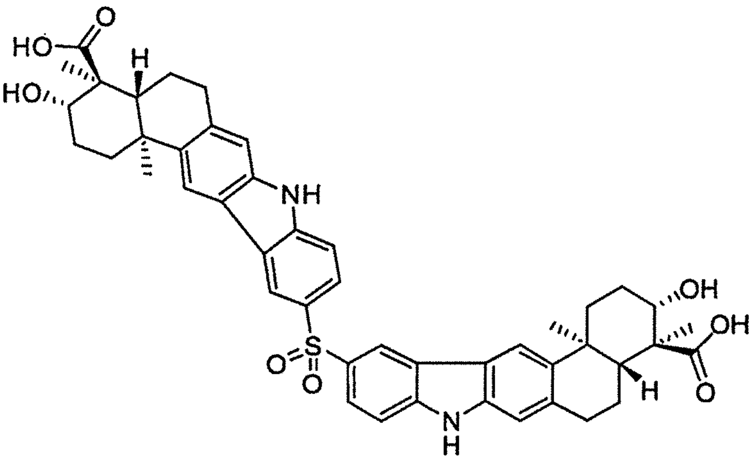

et

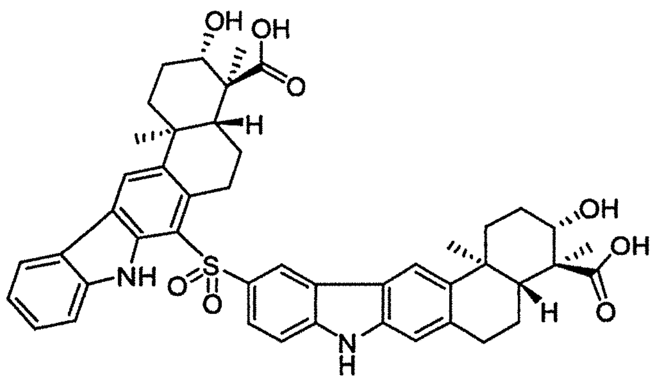

8. Composition pharmaceutique comprenant un composé selon l'une quelconque des revendications 1 à 7 précédentes ou sel pharmaceutiquement acceptable, solvate ou hydrate de celui-ci, éventuellement en combinaison avec un véhicule pharmaceutiquement acceptable.

9. Composé selon l'une quelconque des revendications 1 à 7 ou composition pharmaceutique selon la revendication 8 pour une utilisation dans le traitement des infections bactériennes.

10. Souche de *Streptomyces albus* qui a été déposée auprès de la DSMZ (Inhoffenstr. 7B 38124 Brunswick, Allemagne) par l'Institut de recherche Leibniz sur les substances naturelles et la biologie des infections e. V. - Institut Hans-Knöll (HKI) (Beutenbergstr. 11a, 07745 Iéna, Allemagne), le 20.08.2012 sous le numéro de dépôt DSM26342.

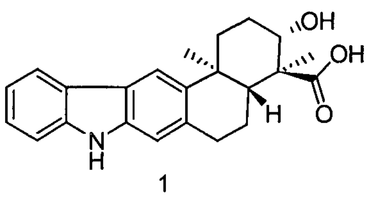

1

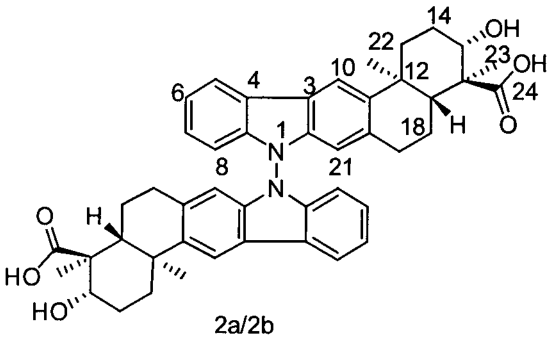

2a/2b

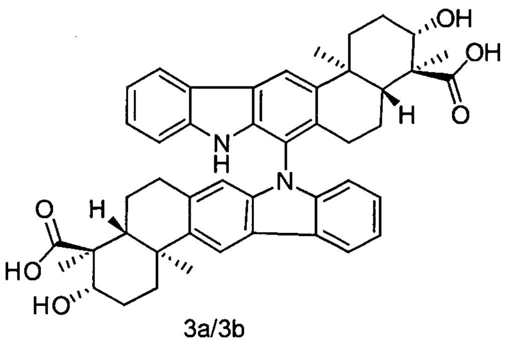

3a/3b

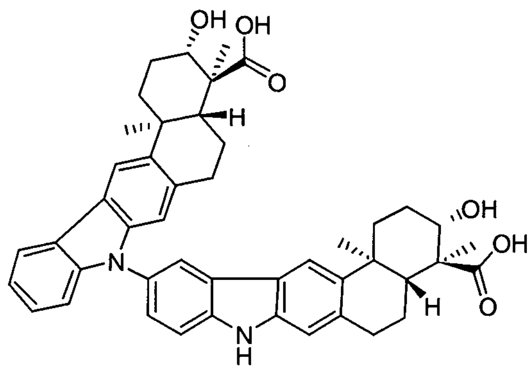

4

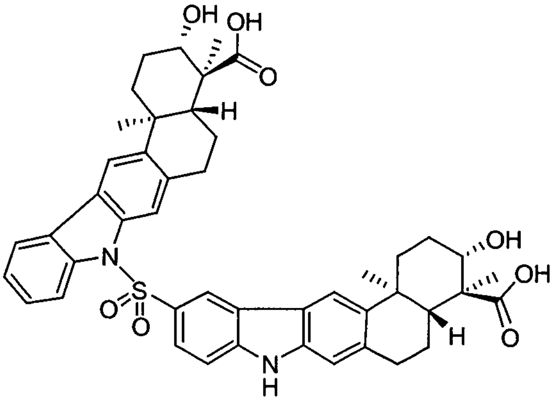

5

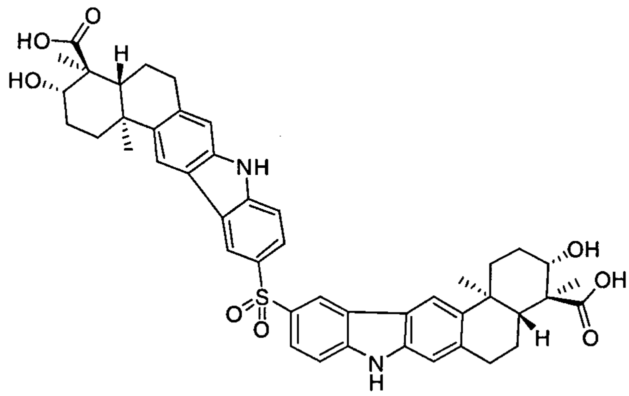

6

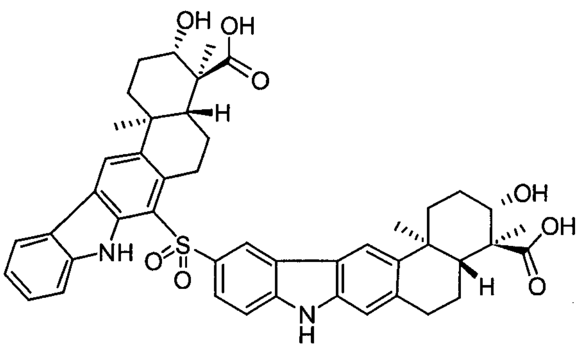

7

**Fig. 1**

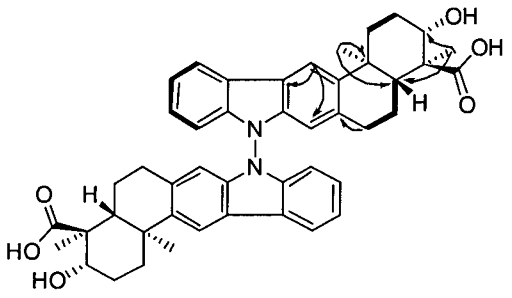

2a/2b

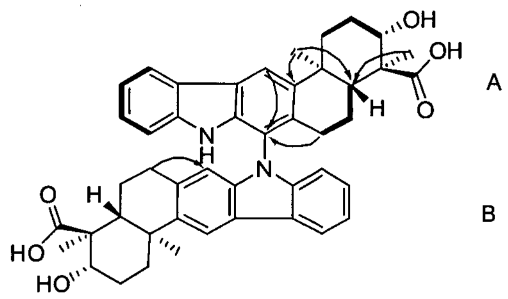

3a/3b

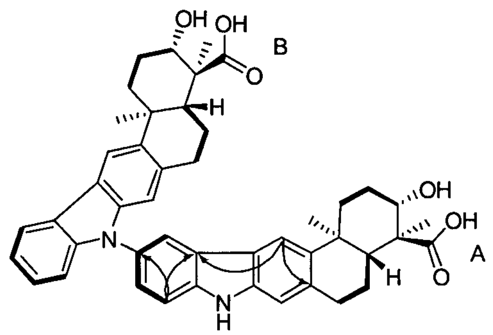

4

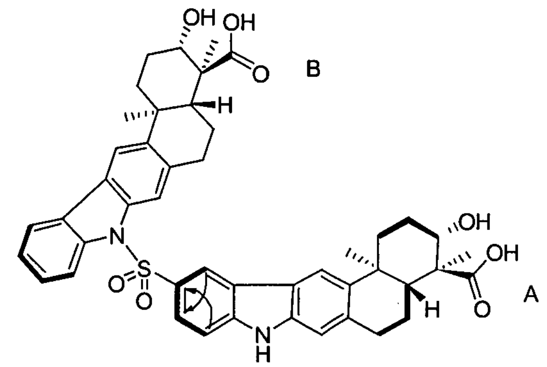

5

COSY

HMBC

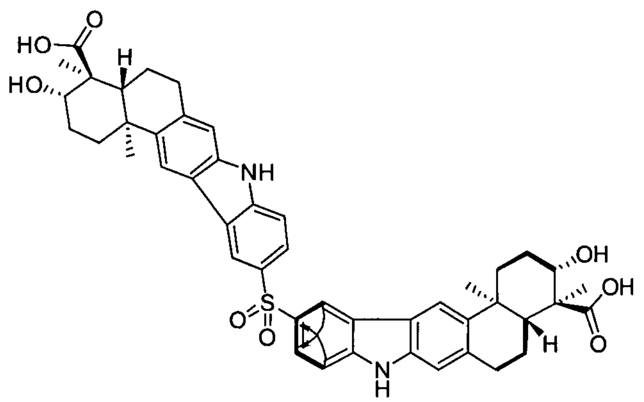

6

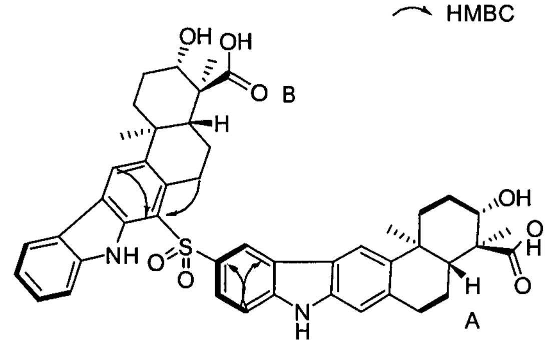

7

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **TAKADA K ; KAJIWARA H ; IMAMURA N.** Oridamycins A and B, anti-Saprolegnia parasitica indolosesquiterpenes isolated from Streptomyces sp. KS84. *J Nat Prod.,* 23 April 2010, vol. 73 (4), 698-701
- **DING L ; MÜNCH J ; GOERLS H ; MAIER A ; FIEBIG HH ; LIN WH ; HERTWECK C.** Xiamycin, a pentacyclic indolosesquiterpene with selective anti-HIV activity from a bacterial mangrove endophyte. *Bioorg Med Chem Lett.,* 15 November 2010, vol. 20 (22), 6685-7
- **DING L ; MAIER A ; FIEBIG HH ; LIN WH ; HERTWECK C.** A family of multicyclic indolosesquiterpenes from a bacterial endophyte. *Org Biomol Chem.,* 07 June 2011, vol. 9 (11), 4029-31
- **LI H ; ZHANG Q ; LI S ; ZHU Y ; ZHANG G ; ZHANG H ; TIAN X ; ZHANG S ; JU J ; ZHANG C.** Identification and characterization of xiamycin A and oxiamycin gene cluster reveals an oxidative cyclization strategy tailoring indolosesquiterpene biosynthesis. *J Am Chem Soc.,* 30 May 2012, vol. 134 (21), 8996-9005